# EUROPEAN PATENT APPLICATION

(11) **EP 3 845 260 A1**
(43) Date of publication of application: **07.07.2021**
(21) Application number: 20796684.7
(22) Date of filing: 18.09.2020
(51) Int. Cl.: A61M 16/04

(54) **THREE-CAVITY FLUSHABLE FISH MOUTH TYPE LARYNGEAL MASK AIRWAY CATHETER**

(30) Priority: 30.10.2019 CN 201911043241
(71) Applicant: Anhui Exploration Medical Devices Technology Co., Ltd., Huainan, Anhui 232200 (CN)
(72) Inventor: FANG, Zhengping, Huainan, Anhui 232200 (CN)
(74) Representative: Osterhoff, Utz
(86) International application number: PCT/CN2020/116258
(87) International publication number: WO 2021/082800

(57) **Abstract**

Disclosed is a three-cavity flushable fish mouth laryngeal mask airway catheter, including a cover, the cover is provided with a respiratory chamber for communicating with a respiratory tract and a digestive chamber for communicating with a digestive tract; the digestive chamber is provided with a digestive opening for communicating the digestive chamber and the digestive tract; a peripheral wall of the digestive chamber is tapered from an end away from the digestive opening to the digestive opening; an edge of the digestive opening includes a plurality of protrusions; and the plurality of protrusions are configured to be close to each other due to a squeezing force during a process of inserting the three-cavity flushable fish mouth laryngeal mask airway catheter into a throat to narrow the digestive opening.

## Description

### CROSS-REFERENCE TO RELATED APPLICATIONS

This application claims priority to Chinese Application No. 201911043241.3, filed on October 30, 2019, entitled "THREE-CAVITY FLUSHABLE FISH MOUTH LARYNGEAL MASK AIRWAY CATHETER", the entire disclosure of which is incorporated herein by reference.

### TECHNICAL FIELD

The present disclosure relates to the technical field of medical devices, in particular to a three-cavity flushable fish mouth laryngeal mask airway catheter.

### BACKGROUND

A laryngeal mask is an artificial airway device that not only allows a patient to breathe spontaneously during operation, but also allows positive pressure ventilation. During the process of using the laryngeal mask, it is necessary to insert the laryngeal mask into the body of patient through the throat. Since details of the throat cannot be seen during operation, an operator needs to insert the laryngeal mask with feel and experience, such that an opening at an end of the laryngeal mask corresponding to the digestive tract is difficult to align with the digestive tract and cannot effectively isolate the respiratory tract and the digestive tract. It is necessary to repeatedly adjust the insertion position and prolongs the operation time, thereby increasing the treatment risk.

### SUMMARY

The main objective of the present disclosure is to provide a three-cavity flushable fish mouth laryngeal mask airway catheter, which aims to allow an opening of a digestive tract corresponding to the three-cavity flushable fish mouth laryngeal mask airway catheter to be quickly and accurately inserted into the digestive tract, thereby reducing operation time.

In order to achieve the above objective, the present disclosure provides a three-cavity flushable fish mouth laryngeal mask airway catheter, including a cover, the cover is provided with a respiratory chamber for communicating with a respiratory tract and a digestive chamber for communicating with a digestive tract; the digestive chamber is provided with a digestive opening for communicating the digestive chamber and the digestive tract; a peripheral wall of the digestive chamber is tapered from an end away from the digestive opening to the digestive opening; an edge of the digestive opening includes a plurality of protrusions; and the plurality of protrusions are configured to be close to each other due to a squeezing force during a process of inserting the three-cavity flushable fish mouth laryngeal mask airway catheter into a throat to narrow the digestive opening.

In an embodiment, the digestive opening is elliptical; and the edge of the digestive opening includes four protrusions, and the four protrusions are respectively provided at four vertex corners of an elliptical inscribed quadrilateral.

In an embodiment, a shape of the digestive opening is a shape of a contour of two superimposed ellipses with overlapping centers and unequal long radii and unequal short radii, and the plurality of protrusions are located at intersections of the two ellipses.

In an embodiment, a thickness of a peripheral wall of the digestive opening is smaller than a thickness of the peripheral wall of a digestive chamber.

In an embodiment, an inside wall of the digestive chamber is provided with blocking ribs.

In an embodiment, the blocking ribs are provided in the digestive chamber close to the digestive opening.

In an embodiment, an inside wall of the digestive chamber is provided with supporting ribs.

In an embodiment, the supporting ribs are distributed on the inside wall of the digestive chamber in a corrugated shape.

In an embodiment, the three-cavity flushable fish mouth laryngeal mask airway catheter further includes a connecting tube, the connecting tube is provided with a respiratory cavity and a digestive cavity; one end of the respiratory cavity is in communication with the respiratory chamber, and another end of the respiratory cavity is configured to be in communication with a breathing device for providing breathing gas; one end of the digestive cavity is in communication with the digestive chamber, and another end of the digestive cavity is in communication with an external environment; the digestive cavity includes a first half cavity and a second half cavity both communicating with the digestive chamber; one side of the digestive chamber shares a peripheral wall with one side of the respiratory chamber; and a circulation hole is provided on a peripheral wall of another side of the digestive chamber.

In an embodiment, the first half cavity, the second half cavity, and the respiratory cavity are parallel to each other.

In technical solutions of the present disclosure, a peripheral wall of the digestive chamber is tapered from an end away from the digestive opening to the digestive opening; an edge of the digestive opening is provided with a plurality of protrusions; and the plurality of protrusions are close to each other due to a squeezing force between the patient's throat and the peripheral wall of the digestive chamber during a process of inserting the three-cavity flushable fish mouth laryngeal mask airway catheter into a throat to narrow the digestive opening. As such, the digestive opening can be aligned with the digestive tract, thereby effectively isolating the respiratory tract and the digestive tract, without the need to repeatedly adjust the insertion position, reducing operation time and reducing the treatment risk.

### BRIEF DESCRIPTION OF THE DRAWINGS

In order to more clearly illustrate the embodiments of the present disclosure, drawings used in the embodiments will be briefly described below. Obviously, the drawings in the following description are only some embodiments of the present disclosure. It will be apparent to those skilled in the art that other figures can be obtained according to the structures shown in the drawings without creative work.
FIG. 1 is a schematic diagram of an overall structure of a three-cavity flushable fish mouth laryngeal mask airway catheter according to an embodiment of the present disclosure.
FIG. 2 is a schematic structural diagram of the three-cavity flushable fish mouth laryngeal mask airway catheter from a first perspective according to an embodiment of the present disclosure.
FIG. 3 is a partial enlarged view of portion A in FIG. 2.
FIG. 4 is a schematic structural diagram of the three-cavity flushable fish mouth laryngeal mask airway catheter from a second perspective according to an embodiment of the present disclosure.
FIG. 5 is a schematic structural diagram of the three-cavity flushable fish mouth laryngeal mask airway catheter from a third perspective according to an embodiment of the present disclosure.
FIG. 6 is a schematic structural diagram of the three-cavity flushable fish mouth laryngeal mask airway catheter from a fourth perspective according to an embodiment of the present disclosure.
FIG. 7 is a schematic structural diagram of the three-cavity flushable fish mouth laryngeal mask airway catheter from a fifth perspective according to an embodiment of the present disclosure.

**Description of reference signs**

| reference sign | name | reference sign | name |
|---|---|---|---|
| 100 | cover | 144 | supporting rib |
| 110 | main body | 145 | circulation hole |
| 120 | airbag | 146 | petal-shaped surface |
| 121 | buffer section | 200 | connecting tube |
| 130 | respiratory chamber | 210 | respiratory cavity |
| 131 | respiratory opening | 220 | digestive cavity |
| 132 | boss | 221 | first half cavity |
| 133 | limiting groove | 222 | second half cavity |
| 140 | digestive chamber | 230 | recess |
| 141 | digestive opening | 240 | limiting section |
| 142 | protrusion | 250 | inflation tube |
| 143 | blocking rib | 260 | receiving groove |

The realization of the objective, functional characteristics, and advantages of the present disclosure are further described with reference to the accompanying drawings.

### DETAILED DESCRIPTION OF THE EMBODIMENTS

The technical solutions of the embodiments of the present disclosure will be described in more detail below with reference to the accompanying drawings. It is obvious that the embodiments to be described are only some rather than all of the embodiments of the present disclosure. All other embodiments obtained by persons skilled in the art based on the embodiments of the present disclosure without creative efforts shall fall within the scope of the present disclosure.

It should be noted that if there is a directional indication (such as up, down, left, right, front, rear...) in the embodiments of the present disclosure, the directional indication is only used to explain the relative positional relationship, movement, etc. of the components in a certain posture (as shown in the drawings). If the specific posture changes, the directional indication will change accordingly.

Besides, the descriptions associated with, e.g., "first" and "second," in the present disclosure are merely for descriptive purposes, and cannot be understood as indicating or suggesting relative importance or impliedly indicating the number of the indicated technical feature. Therefore, the feature associated with "first" or "second" can expressly or impliedly include at least one such feature. The meaning of "and/or" appearing in the disclosure includes three parallel scenarios. For example, "A and/or B" includes only A, or only B, or both A and B. In addition, the technical solutions between the various embodiments can be combined with each other, but they must be based on the realization of those of ordinary skill in the art. When the combination of technical solutions is contradictory or cannot be achieved, it should be considered that such a combination of technical solutions does not exist, nor is it within the scope of the present disclosure.

The present disclosure provides a three-cavity flushable fish mouth laryngeal mask airway catheter.

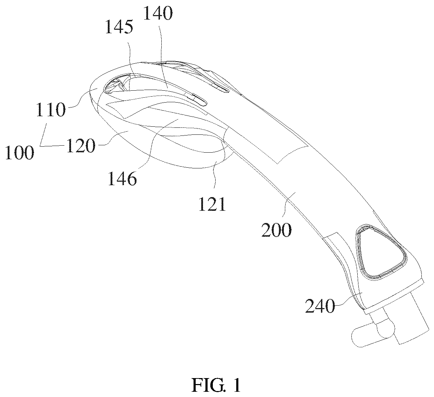

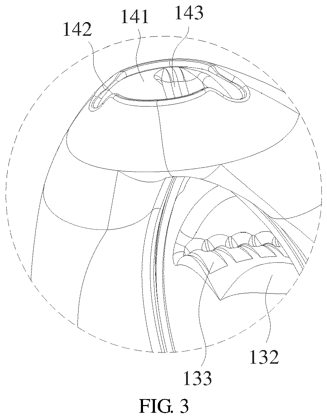

In an embodiment of the present disclosure, as shown in FIG. 1, FIG. 2 and FIG. 3, the three-cavity flushable fish mouth laryngeal mask airway catheter includes a cover 100. The cover 100 is provided with a respiratory chamber 130 for communicating with a respiratory tract and a digestive chamber 140 for communicating with a digestive tract. The digestive chamber 140 is provided with a digestive opening 141 for communicating the digestive chamber 140 and the digestive tract. An inspection device can enter the digestive tract through the digestive chamber 140 and the digestive opening 141 and check the patient.

A peripheral wall of the digestive chamber 140 is tapered from an end away from the digestive opening 141 to the digestive opening 141. An edge of the digestive opening 141 is provided with a plurality of protrusions 142. The plurality of protrusions 142 are configured to be close to each other due to a squeezing force during a process of inserting the three-cavity flushable fish mouth laryngeal mask airway catheter into a throat to narrow the digestive opening 141.

In this embodiment, when the three-cavity flushable fish mouth laryngeal mask airway catheter is inserted into the patient's throat, the patient's throat and the peripheral wall of the digestive chamber 140 generate a mutual squeezing force, such that the plurality of protrusions 142 are close to each other to narrow the digestive opening 141, thereby the digestive opening 141 can be better aligned with the digestive tract. When the edge of the peripheral wall of the digestive chamber 140 enters an airway opening, the digestive opening 141 expands until the peripheral wall of the digestive chamber 140 and the inner wall of the airway opening form a seal, such that the respiratory tract and the digestive tract are effectively isolated without repeated adjustment of the insertion position, the operation time is reduced, and the treatment risk is reduced.

In an embodiment, the digestive opening 141 is elliptical; and the edge of the digestive opening is provided with four protrusions 142, and the four protrusions 142 are respectively provided at four vertex corners of an elliptical inscribed quadrilateral. When the three-cavity flushable fish mouth laryngeal mask airway catheter is inserted into the patient's throat, the patient's throat and the peripheral wall of the digestive chamber 140 generate a mutual squeezing force, the four protrusions 142 move closer to the center of the ellipse, to narrow the digestive opening 141, so that the digestive opening 141 can be better aligned with the digestive tract. Since the peripheral wall of the digestive chamber 140 is tapered from the end away from the digestive opening 141 to the digestive opening 141, the four protrusions 142 are more likely to move closer to the center of the ellipse, which avoids unsmooth insertion due to rolling of the protrusions 142 during the insertion of the three-cavity flushable fish mouth laryngeal mask airway catheter.

In another embodiment, a shape of the digestive opening 141 is a shape of a contour of two superimposed ellipses with overlapping centers and unequal long radii and unequal short radii. The two ellipses intersect at four points, and the four protrusions 142 are respectively provided at the four points. When the three-cavity flushable fish mouth laryngeal mask airway catheter is inserted into the patient's throat, the patient's throat and the peripheral wall of the digestive chamber 140 generate a mutual squeezing force, the four protrusions 142 move closer to the center of the ellipse, to narrow the digestive opening 141, so that the digestive opening 141 can be better aligned with the digestive tract.

In other embodiments, the shape of the digestive opening 141 and the number and position of the protrusions 142 can be adjusted adaptively.

In an embodiment, a thickness of a peripheral wall of the digestive opening 141 is smaller than a thickness of the peripheral wall of a digestive chamber 140, such that the digestive opening 141 is easier to deform and narrow the opening during the insertion process, which is more convenient for the smooth insertion of the three-cavity flushable fish mouth laryngeal mask airway catheter.

In an embodiment, an inside wall of the digestive chamber 140 is provided with blocking ribs 143. When the digestive opening 141 is narrowed due to the squeezing force during the insertion process, the inner side of the peripheral wall of the digestive opening 141 may fit together due to the squeezing force. However, the three-cavity flushable fish mouth laryngeal mask airway catheter is usually made of a material with a smooth surface, and the inner side of the peripheral wall of the digestive opening 141 is difficult to separate after being stuck together due to the squeezing force. As a result, the digestive opening 141 may be closed or the digestive opening 141 may be changed after the insertion of the three-cavity flushable fish mouth laryngeal mask airway catheter, which may result in an unsmooth treatment process.

In an embodiment, the blocking ribs 143 are provided in the digestive chamber 140 close to the digestive opening 141. Since the peripheral wall of the digestive chamber 140 is tapered from the end away from the digestive opening 141 to the digestive opening 141, the peripheral wall of the digestive opening 141 is more prone to fit compared to the peripheral walls of other parts of the digestive chamber 140. Arranging the blocking ribs 143 in the digestive chamber 140 close to the digestive opening 141 enables the blocking ribs 143 to function more effectively, further reducing the probability that the digestive opening 141 will be closed after the insertion of the three-cavity flushable fish mouth laryngeal mask airway catheter.

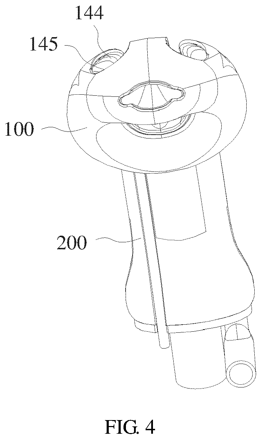

As shown in FIG. 1 and FIG. 4, in an embodiment, an inside wall of the digestive chamber 140 is provided with supporting ribs 144, such that after the three-cavity flushable fish mouth laryngeal mask airway catheter is inserted, the digestive chamber 140 can better maintain a full and smooth state, which is more conducive to the smooth progress of the treatment process.

In an embodiment, the supporting ribs 144 are distributed on the inside wall of the digestive chamber 140 in a corrugated shape, such that the inside wall of the digestive chamber 140 can be supported everywhere, thereby the three-cavity flushable fish mouth laryngeal mask airway catheter better maintains a full and smooth state, the strength of the peripheral wall of the digestive chamber 140 can be increased, and the peripheral wall of the digestive chamber 140 is not easy to rupture.

In another embodiment, the supporting ribs 144 are distributed on the inside wall of the digestive chamber 140 in a fish scale shape, so that the peripheral wall of the digestive chamber 140 is supported more uniformly, thereby the digestive chamber 140 can better maintain the preset shape.

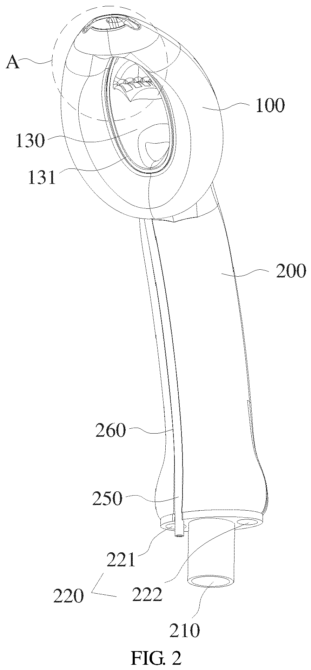

As shown in FIG. 1 and FIG. 2, in an embodiment, the three-cavity flushable fish mouth laryngeal mask airway catheter further includes a connecting tube 200. The connecting tube 200 is provided with a respiratory cavity 210 and a digestive cavity 220. One end of the respiratory cavity 210 is in communication with the respiratory chamber 130, and another end of the respiratory cavity 210 is in communication with a breathing device for providing breathing gas. One end of the digestive cavity 220 is in communication with the digestive chamber 140, and another end of the digestive cavity 220 is in communication with an external environment. The breathing device inputs breathing gas to the respiratory tract through the connecting tube 200, so that the patient can breathe spontaneously during the treatment. In addition, the gas input into the throat or respiratory cavity 210 may be anesthetic gas, oxygen-rich gas, or active ventilation gas.

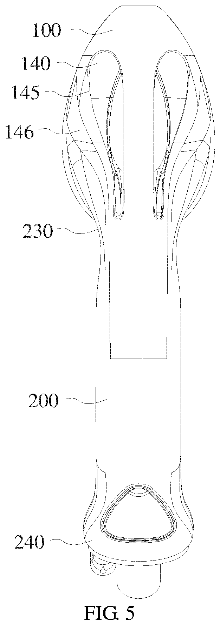

As shown in FIG. 5, in an embodiment, a transition section is provided at one end of the connecting tube 200 connected to the cover 100. The transition section has a recess 230 with an outer diameter smaller than an outer diameter of the connecting tube 200. When the operator inserts the three-cavity flushable fish mouth laryngeal mask airway catheter into the patient's throat, the connecting tube 200 and the patient's throat are squeezed against each other, and the squeezing force at the epiglottis is relatively large. In the previous stage of the insertion of the three-cavity flushable fish mouth laryngeal mask airway catheter, each section of the connecting tube 200 with the same outer diameter remains stable against the epiglottis. Since the outer diameter of the recess 230 is smaller than the outer diameter of the connecting tube 200, when the recess 230 passes through the epiglottis, the squeezing force on the epiglottis suddenly decreases. That is, during the process of inserting the three-cavity flushable fish mouth laryngeal mask airway catheter into the patient's throat, the recess 230 will fall when it passes through the epiglottis, such that the operator can accurately know the insertion position of the three-cavity flushable fish mouth laryngeal mask airway catheter without seeing the details of the throat, reducing the number of times to adjust the insertion position.

In an embodiment, after the transition section is smoothly tapered from the end connected to the cover 100 to form a recess 230, it then smoothly expands until the outer diameter of the transition section is equal to the outer diameter of the connecting tube 200. Therefore, while keeping the recess fall during the insertion process, the process of inserting and removing the three-cavity flushable fish mouth laryngeal mask airway catheter is smoother, and the discomfort caused to the patient is reduced.

In an embodiment, the outer side wall of the cover 100 is provided with a plurality of petal-shaped surfaces 146, adjacent petal-shaped surfaces 146 intersect, and the intersection line of the plurality of petal-shaped surfaces 146 plays a role of positioning on the outer surface of the cover 100, such that the three-cavity flushable fish mouth laryngeal mask airway catheter will not move randomly after being inserted into the patient's throat, thus making the treatment process more stable. An intersection line between the adjacent petal-shaped surfaces 146 smoothly extends from an end of the cover 100 close to the connecting tube 200 to an end of the cover 100 away from the connecting tube 200. That is, an extension direction of the intersection line between the adjacent petal-shaped surfaces 146 is consistent with the insertion direction of the three-cavity flushable fish mouth laryngeal mask airway catheter, such that the petal-shaped surface 146 can not only prevent the three-cavity flushable fish mouth laryngeal mask airway catheter from moving randomly, but also does not hinder the insertion of the three-cavity flushable fish mouth laryngeal mask airway catheter.

It should be understood that, in this embodiment, the intersection of the adjacent petal-shaped surfaces 146 is a rounded ridge-like structure, rather than a blade-like structure.

In an embodiment, the cover 100 includes a front side towards the respiratory tract, a rear side away from the respiratory tract, an insertion end inserted into the throat, and a connecting end away from the insertion end. The connecting end is connected to the connecting tube 200, the respiratory chamber 130 is located on the front side, and the digestive chamber 140 is located on the rear side. One side of the respiratory chamber 130 shares a side wall with one side of the digestive chamber 140, on the one hand, which saves the materials required for making the cover 100, on the other hand, which reduces the volume of the cover 100 and makes the cover 100 more flexible, thereby making the patient more comfortable.

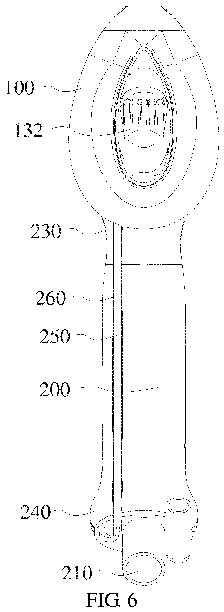

As shown in FIG. 2, FIG. 3 and FIG. 6, the front side is provided with a respiratory opening 131 for connecting the respiratory chamber 130 with the respiratory tract. The rear side is arched. A boss 132 is provided in the respiratory chamber 130. The boss 132 is located on the side wall shared by the respiratory chamber 130 and the digestive chamber 140. The boss 132 has an inclined surface towards the respiratory cavity 210 and the respiratory opening 131. When the three-cavity flushable fish mouth laryngeal mask airway catheter is inserted into the patient's throat, the cover 100 deforms under the squeezing force of the throat. The end of the cover 100 first inserted into the throat is brought closer to the arcuate recess, so that the shape of the respiratory opening 131 is changed, and the boss 132 is driven to approach the plane the respiratory opening 131 is located. The cover 100 fits more closely to the opening of the respiratory tract, reduces air dead space, and facilitates the circulation of fresh air. The setting of the boss 132 enables the operator to insert the inspection device into the respiratory tract through the respiratory chamber 130 during the treatment process, the inclined surface of the boss 132 can guide the inspection device, so that the inspection device can be inserted into the respiratory tract more accurately, and reduce the probability of the inspection device poking the wall of the respiratory tract.

In an embodiment, one side of the boss 132 close to the respiratory cavity 210 and the side wall shared by the respiratory chamber 130 and the digestive chamber 140 smoothly transitions. While the boss 132 plays a guiding role for the inserted inspection device, the inspection device can pass through the connection between the boss 132 and the side wall of the respiratory cavity 210 more smoothly, so that the inspection device can be inserted more smoothly.

In an embodiment, an edge of the boss 132 away from the respiratory cavity 210 is rounded. When the boss 132 is driven by the deformed cover 100 to approach the plane the respiratory opening 131 is located, the boss 132 may contact the throat. The rounded corner provided on the edge of the boss 132 makes the boss 132 contact with the throat, the irritation of the boss 132 to the throat is reduced, thereby reducing the discomfort caused to the patient by the three-cavity flushable fish mouth laryngeal mask airway catheter.

In an embodiment, the boss 132 is provided with a limiting groove 133 extending from the respiratory cavity 210 to the respiratory opening 131. When the inspection device is inserted from the respiratory cavity 210, the limiting groove 133 can guide and limit the inspection device, such that the inspection device can be inserted into the required position more accurately, and the inspection device is not easy to move randomly after insertion, to make the treatment process smoother.

In an embodiment, a plurality of limiting grooves 133 are provided on the boss 132 at intervals, so that there are more choices for the insertion position of the inspection device, so that the operator can select different limiting grooves 133 according to actual needs.

In an embodiment, a cross section of the limiting groove 133 is arc-shaped. Since the inspection device usually includes a cylindrical tubular structure, the limiting groove 133 with an arc-shaped cross section can fit the inspection device more closely, so that the limiting groove 133 has a better guiding and limiting effect on the inspection device.

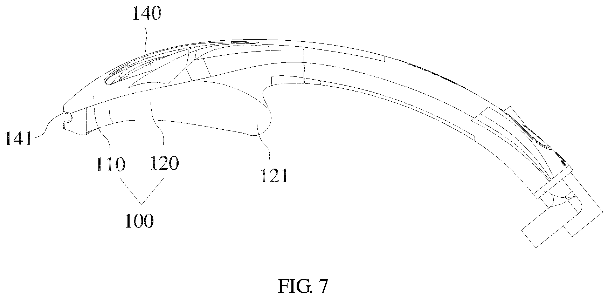

As shown in FIG. 2 and FIG. 7, in an embodiment, the cover 100 includes a main body 110 and an airbag 120. The main body 100 includes a front side towards the respiratory tract, a rear side away from the respiratory tract, an insertion end inserted into the throat, and a connecting end away from the insertion end. The main body 110 is provided with a respiratory chamber 130 for communicating with the respiratory tract and a digestive chamber 140 for communicating with the digestive tract. The respiratory chamber 130 is located on the front side. The digestive chamber 140 is located on the rear side. The front side is provided with a respiratory opening 131 for communicating the respiratory chamber 130 and the respiratory tract. During the treatment, the connecting tube 200 is connected to a respiratory device for providing breathing gas, and the breathing gas is provided to the respiratory chamber 130 through the connecting tube 200, so that the patient can breathe independently during the treatment. It should be understood that the airbag 120 is provided on the front side of the cover 100. The front side of the main body 110 is located on the front side of the cover 100. The rear side of the main body 110 is also the rear side of the cover 100. The insertion end of the main body 110 corresponds to the insertion end of the cover 100. The connecting end of the body 110 corresponds to the connecting end of the cover 100.

The airbag 120 is provided around the edge of the respiratory opening 131 to form a seal with the patient's airway opening during treatment, thereby breathing gas enters the patient's respiratory tract smoothly, while other undesirable gases cannot enter the patient's respiratory tract, making the patient more comfortable and safer. One side of the airbag 120 is fixed to the edge of the respiratory opening 131, and another side of the airbag 120 is offset towards the extension direction of the connecting tube 200 at the connecting end, so that the airbag 120 forms an acute angle with the connecting tube 200 at the connecting end. Since the airbag 120 forms an acute angle with the connecting tube 200 at the connecting end, when the airbag 120 is squeezed, the airbag 120 is deformed under the squeezing force. The airbag 120 is close to the connecting tube 200, and the angle formed between the airbag 120 and the connecting tube 200 is reduced. However, when the squeezing force received by the airbag 120 becomes smaller, the airbag 120 recovers to deform so that the angle formed between the airbag 120 and the connecting tube 200 increases.

In this embodiment, when the three-cavity flushable fish mouth laryngeal mask airway catheter is inserted into the patient's throat, the airbag 120 forms a seal with the patient's airway opening, and the tissue of the patient's airway opening and the airway 120 are squeezed against each other. On one hand, the airbag 120 can form a seal with the patient's respiratory tract under the action of the gas filled inside to ensure the smooth progress of the treatment process. On the other hand, the airbag 120 can more flexibly adjust the angle with the connecting tube 200 according to the mutual squeezing force between the tissue of the patient's airway opening and the airbag 120, such that the squeezing force received by the tissue of the patient's airway opening can be more stably maintained within a preset range, which relieves the squeezing of the patient's tissue by the three-cavity flushable fish mouth laryngeal mask airway catheter and reduces the patient's discomfort.

Besides, in this embodiment, since the airbag 120 has the characteristic of being able to adjust the angle with the connecting tube 200 according to the mutual squeezing force between the tissue of the patient's airway opening and the airbag 120, when the operator inserts the three-cavity flushable fish mouth laryngeal mask airway catheter into the patient's throat, the airbag 120 prevents the insertion process from obstructing the insertion process and makes the insertion process smoother.

In an embodiment, the airbag 120 is tapered from its middle section to the connecting end, and the airbag 120 forms an arc-shaped buffer section 121 at the connecting end. Therefore, when the cover 100 leaves the patient's throat, the reduced connecting end first contacts the outlet of the throat. The airbag 120 can leave the throat more smoothly, and the arc-shaped buffer section 121 makes the airbag 120 less squeeze and stimulate the throat when it leaves the throat. When the airbag 120 leaves the throat, the reduced connecting end first leaves the outlet of the throat, and the middle section of the airbag 120 is larger. Under the squeezing of the throat, the airbag 120 becomes slender and easier to leave the throat.

In an embodiment, the side of the buffer section 121 away from the main body 110 is offset towards the extending direction of the connecting tube 200, and the buffer section 121 forms an acute angle with the connecting tube 200 at the connecting end. During the insertion of the three-cavity flushable fish mouth laryngeal mask airway catheter, the buffer section 121 plays a buffering function, which not only maintains the sealing effect, but also relieves the squeezing of the patient's tissues from the three-cavity flushable fish mouth laryngeal mask airway catheter.

In an embodiment, the airbag 120 has a ring shape, and the shape of the airbag 120 is adapted to the shape of the respiratory opening 131. When the three-cavity flushable fish mouth laryngeal mask airway catheter is inserted into the patient's throat, the airbag 120 is located between the respiratory opening 131 and the patient's airway opening. The airbag 120 just shields the gap between the respiratory opening 131 and the patient's airway opening, so that the sealing effect is better.

In an embodiment, the smooth transition between the airbag 120 and the connecting tube 200 makes the three-cavity flushable fish mouth laryngeal mask airway catheter inserted into the patient's throat, the inflated airbag 120 can also be inserted more smoothly, reducing the discomfort to the patient.

In an embodiment, an inflation tube 250 is provided on the airbag 120. The outer side wall of the connecting tube 200 is provided with a receiving groove 260 for receiving the inflation tube 250. Receiving the inflation tube 250 in the receiving groove 260 can not only inflate the airbag 120 so that the airbag 120 has a sealing effect, but also prevent the inflation tube 250 from hindering the treatment process.

In an embodiment, limiting protrusions are provided on both sides of the receiving groove 260. The inflation tube 250 is elastically clamped with the limiting protrusions on both sides of the receiving groove 260. On one hand, the inflatable tube 250 is more stably stored in the receiving groove 260 to prevent the inflatable tube 250 from hindering the treatment process. On the other hand, the elastic clamping makes the inflation tube 250 difficult to be damaged, so as to better avoid the air leakage problem.

In an embodiment, the end of the inflation tube 250 connected to the airbag 120 is provided on the side of the airbag 120 close to the main body 110, such that the shortest distance between the connection of the inflation tube 250 and the airbag 120 and the receiving groove 260 is made shorter, and the inflation tube 250 is more fully stored in the receiving groove 260.

In an embodiment, the end of the connecting tube 200 away from the cover 100 is provided with a limiting section 240. An outer diameter of the limiting section 240 is greater than an outer diameter of the connecting tube 200. After the three-cavity flushable fish mouth laryngeal mask airway catheter is inserted into the patient's throat, in order to make the treatment process more smoothly, the end of connecting tube 200 of the three-cavity flushable fish mouth laryngeal mask airway catheter is usually fixed near the patient's lips with tape. The outer diameter of the limiting section 240 is greater than the outer diameter of the connecting tube 200, so that the connection between the limiting section 240 and the connecting tube 200 forms a concave structure, which makes it easier to wrap the tape and make the tape not easy to loosen.

In an embodiment, the limiting section 240 and the connecting tube 200 smoothly transition, so as to better fit the tape.

In an embodiment, the digestive cavity 220 includes a first half cavity 221 and a second half cavity 222 both communicating with the digestive chamber 140. One side of the digestive chamber 140 shares a peripheral wall with one side of the respiratory chamber 130; and a circulation hole 145 is provided on a peripheral wall of another side of the digestive chamber 140. During the treatment, breathing gas enters the respiratory chamber 130 through the respiratory cavity 210 to provide breathing gas for the patient. The first half cavity 221 is connected to a suction source and the second half cavity 222 is connected to a positive pressure source or communicates with the atmosphere. Alternatively, the second half cavity 222 is connected to a suction source and the first half cavity 221 is connected to a positive pressure source or communicates with the atmosphere. Therefore, during the treatment process, waste fluids such as saliva, gastric juice and blood produced by the patient first enter the digestive chamber 140 through the digestive opening 141 under the action of the suction source, then are discharged from the patient's body through the first half cavity 221 or the second half cavity 222 communicating with the digestive chamber 140, thereby effectively preventing the waste liquid generated during the treatment of the patient from entering the airway and causing discomfort to the patient.

In an embodiment, the first half cavity 221, the second half cavity 222 and the respiratory cavity 210 are parallel to each other, such that the cavity wall of the first half cavity 221, the cavity wall of the second half cavity 222, and the cavity wall of the respiratory cavity 210 have uniform thickness everywhere in the length direction of the connecting tube 200, so that the connecting tube 200 has a more stable structure and is not easy to break.

In an embodiment, a cross section of the connecting tube 200 is oval, that is, the connecting tube 200 is a flat tube. On the same cross section of the connecting tube 200, a center of the respiratory cavity 210 coincides with a center of the ellipse. The first cavity and the second cavity are symmetrically arranged on both sides of the respiratory cavity 210. The cover 100 is also flat, and the digestive opening 141 is also oval. That is, a shape of the digestive opening 141 is adapted to the distribution positions of the first cavity and the second cavity, so that the waste liquid can be discharged more quickly.

In an embodiment, the connecting tube 200 has an arc shape from an end close to the cover 100 to an end far away from the cover 100, such that the connecting tube 200 is more easily inserted into the patient's throat and has a smaller squeezing force on the patient's throat.

The above are only some embodiments of the present disclosure, and do not limit the scope of the present disclosure thereto. Under the inventive concept of the present disclosure, equivalent structural transformations made according to the description and drawings of the present disclosure, or direct/indirect application in other related technical fields are included in the scope of the present disclosure.

## Claims

1. A three-cavity flushable fish mouth laryngeal mask airway catheter, comprising a cover, wherein:
the cover is provided with a respiratory chamber for communicating with a respiratory tract and a digestive chamber for communicating with a digestive tract;
the digestive chamber is provided with a digestive opening for communicating the digestive chamber and the digestive tract;
a peripheral wall of the digestive chamber is tapered from an end away from the digestive opening to the digestive opening;
an edge of the digestive opening comprises a plurality of protrusions; and
the plurality of protrusions are configured to be close to each other due to a squeezing force during a process of inserting the three-cavity flushable fish mouth laryngeal mask airway catheter into a throat to narrow the digestive opening.

2. The three-cavity flushable fish mouth laryngeal mask airway catheter of claim 1, wherein:
the digestive opening is elliptical; and
the edge of the digestive opening comprises four protrusions, and the four protrusions are respectively provided at four vertex corners of an elliptical inscribed quadrilateral.

3. The three-cavity flushable fish mouth laryngeal mask airway catheter of claim 1, wherein:
a shape of the digestive opening is a shape of a contour of two superimposed ellipses with overlapping centers and unequal long radii and unequal short radii, and
the plurality of protrusions are located at intersections of the two ellipses.

4. The three-cavity flushable fish mouth laryngeal mask airway catheter of claim 1, wherein a thickness of a peripheral wall of the digestive opening is smaller than a thickness of the peripheral wall of a digestive chamber.

5. The three-cavity flushable fish mouth laryngeal mask airway catheter of claim 1, wherein an inside wall of the digestive chamber is provided with blocking ribs.

6. The three-cavity flushable fish mouth laryngeal mask airway catheter of claim 5, wherein the blocking ribs are provided in the digestive chamber close to the digestive opening.

7. The three-cavity flushable fish mouth laryngeal mask airway catheter of claim 1, wherein an inside wall of the digestive chamber is provided with supporting ribs.

8. The three-cavity flushable fish mouth laryngeal mask airway catheter of claim 7, wherein the supporting ribs are distributed on the inside wall of the digestive chamber in a corrugated shape.

9. The three-cavity flushable fish mouth laryngeal mask airway catheter of claim 1, further comprising a connecting tube, wherein:
the connecting tube is provided with a respiratory cavity and a digestive cavity;
one end of the respiratory cavity is in communication with the respiratory chamber, and another end of the respiratory cavity is configured to be in communication with a breathing device for providing breathing gas;
one end of the digestive cavity is in communication with the digestive chamber, and another end of the digestive cavity is in communication with an external environment;
the digestive cavity comprises a first half cavity and a second half cavity both communicating with the digestive chamber;
one side of the digestive chamber shares a peripheral wall with one side of the respiratory chamber; and
a circulation hole is provided on a peripheral wall of another side of the digestive chamber.

10. The three-cavity flushable fish mouth laryngeal mask airway catheter of claim 9, wherein the first half cavity, the second half cavity, and the respiratory cavity are parallel to each other.
